# EUROPÄISCHE PATENTSCHRIFT

(11) **EP 3 034 068 B1**
(45) Veröffentlichungstag und Bekanntmachung des Hinweises auf die Patenterteilung: **10.04.2019**
(21) Anmeldenummer: 15192559.1
(22) Anmeldetag: 02.11.2015
(51) Int. Cl.: A61K 8/92, A61Q 5/08, A61K 8/03

(54) **3-KOMPONENTEN-HAARAUFHELLUNGSMITTEL**
3-COMPONENT HAIR BRIGHTENING AGENT
AGENT D'ECLAIRCISSEMENT DES CHEVEUX A 3 COMPOSANTS

(30) Priorität: 19.12.2014 DE 102014226531
(43) Veröffentlichungstag der Anmeldung: 22.06.2016
(73) Patentinhaber: Henkel AG & Co. KGaA, 40589 Düsseldorf (DE)
(72) Erfinder: REICHERT, Anja, 40629 Düsseldorf (DE); BABIEL, Sabine, 47447 Moers (DE); GÜNTHER, Katja, 40721 Hilden (DE)

(56) Entgegenhaltungen:
- EP-A2- 2 345 400
- WO-A1-2004/055151
- FR-A1- 3 012 332
- US-A1- 2013 061 866

## Beschreibung

Die vorliegende Anmeldung betrifft Zusammensetzungen, die sich als Alkalisierungszubereitung für 3-Komponenten-Haaraufhellungsmittel eignen, die neben der Alkalisierungszubereitung eine Oxidationsmittelzubereitung in Form einer wässrigen Wasserstoffperoxidzusammensetzung und eine wasserfreie, pulverförmige Oxidationsmittelzubereitung, enthaltend eine feste Peroxidverbindung, umfassen.

Ein weiterer Gegenstand der vorliegenden Anmeldung ist eine Mehrkomponentenverpackungseinheit (Kit) zur verbesserten Aufhellung und/oder Blondierung von keratinischen Fasern, insbesondere menschlichen Haaren. Weiterhin wird ein Verfahren zur Aufhellung von keratinischen Fasern unter Verwendung der genannten Alkalisierungszubereitung und des diese Alkalisierungszubereitung umfassenden Kits beschrieben.

Das Aufhellen der eigenen Haarfarbe ist seit jeher der Wunsch vieler Verbraucher, da eine blonde Haarfarbe als attraktiv und in modischer Hinsicht erstrebenswert betrachtet wird. Für diesen Zweck sind im Markt verschiedene Blondiermittel mit unterschiedlicher Blondierleistung erhältlich. Die in diesen Produkten enthaltenen Oxidationsmittel sind in der Lage, durch die oxidative Zerstörung des haareigenen Farbstoffes Melanin die Haarfaser aufzuhellen. Für einen moderaten Blondiereffekt genügt der Einsatz von Wasserstoffperoxid - gegebenenfalls unter Einsatz von Ammoniak oder anderen Alkalisierungsmitteln - als Oxidationsmittel allein. Für das Erzielen eines stärkeren Blondiereffekts wird üblicherweise eine Mischung aus Wasserstoffperoxid und Peroxodisulfatsalzen und/oder Peroxomonosulfatsalzen eingesetzt. Zur Verstärkung der Blondierwirkung enthalten die Mittel höhere Einsatzkonzentrationen an Wasserstoffperoxid und Persalzen, insbesondere Persulfaten. Dunkles, dunkelbraunes oder schwarzes Haar lässt sich so in einem Schritt um 4 bis 6 Nuancen aufhellen. Das Wasserstoffperoxid und die Persalze werden bis zur Anwendung getrennt voneinander aufbewahrt, um die Persalze nicht vorzeitig zu deaktivieren. Die Wasserstoffperoxid-Komponente weist zur Stabilisierung des Wasserstoffperoxids einen sauren pH-Wert, insbesondere einen pH-Wert von pH 2,5 bis pH 5,5, insbesondere von pH 3 bis pH 5, auf, jeweils gemessen bei 20°C.

Um die Aktivität des Wasserstoffperoxids zu verbessern und die Blondierwirkung auf der keratinischen Faser zu verstärken, ist es jedoch vorteilhaft, wenn die Anwendungsmischung aus Wasserstoffperoxid und Persalz einen alkalischen pH-Wert besitzt. Daher wird die Anwendungsmischung aus Wasserstoffperoxid und Persalz mit einer Alkalisierungsmittelkomponente kombiniert.

Setzt man der Alkalisierungsmittelkomponente Oxidationsfarbstoffvorprodukte und/oder direktziehende Farbstoffe zu, so kann das Haar gleichzeitig gefärbt werden. Entsprechende 3-Komponenten-Haarfärbemittel werden insbesondere für Verbraucher mit sehr dunklem, Melanin-reichem Haar, angeboten.

Mit der Aufhellung geht jedoch auch eine Schädigung des Haares einher, da nicht nur die Farbstoffe des Haares, sondern auch die übrigen Strukturbestandteile des Haares oxidativ geschädigt werden. Je nach Ausprägung des Schädigungsgrades reicht dieser von rauem, sprödem und schwieriger auskämmbarem Haar über eine verminderte Widerstandsfähigkeit und Reißfestigkeit des Haares bis hin zu Haarbruch. Je größer die Menge des eingesetzten Wasserstoffperoxids und gegebenenfalls der Peroxodisulfate ist, desto stärkere Schädigungen werden daher in der Regel auf den Keratinfasern hervorgerufen.

Um die Haarschädigung zu minimieren und den schädigenden Effekt der Oxidationsmittel auszugleichen, wird immer wieder versucht, Persalz-haltige Haaraufhellungs- und -färbemittel mit einem höheren Gehalt an Ölen zu formulieren.

Im Stand der Technik sind beispielsweise Blondiermittelsuspensionen beschrieben, die wasserfreie Suspensionen von feinteiligen festen Peroxidverbindungen in einem Öl oder einer Ölmischung darstellen, die gegebenenfalls mit einem Ölgeliermittel verdickt sein kann, siehe EP 0778020, EP 1034777 und EP 1380287. Nachteilig hierbei ist, dass das Herstellen einer homogenen Mischung aus dieser sehr hydrophoben Paste und der stark wasserhaltigen Wasserstoffperoxidzubereitung sowie der ebenfalls wasserhaltigen Alkalisierungsmittelkomponente schwierig ist und länger andauerndes, kräftiges Schütteln erfordert. Auch ist die Herstellung einer Blondiermittelsuspension technisch aufwändiger als die Herstellung einer pulverförmigen Persalzmischung.

US 2013/0061866 A1 offenbart eine Haarfärbezusammensetzung, die einen Farbstoff, eine hydrophobe Phase, eine hydrophile Phase, ein Tensid sowie ein spezielles Verdickungsmittel enthält. Laut US 2013/0061866A1 werden zweischichtige Zusammensetzungen nur dann erhalten, wenn zwei hydrophile Färbezusammensetzungen, die jeweils ein Assoziativpolymer als Verdickungsmittel enthalten, miteinander gemischt werden. Kombinationen aus einem nichtionischen Tensid und einem anionischen Tensid in Form von Alkylsulfaten, Alkylethersulfaten oder Alkylethercarbonsäuren sind nicht offenbart. EP 2345400A2 offenbart eine einphasige Emulsion C2, die ein Alkalisierungsmittel (Monoethanolamin), 11,4 Gew.-% eines Öls (Dibutyl Adipat, Decyl Oleat) und 0,85 bis 1,7 Gew.-% eines Alkylsulfats enthält. Weiterhin offenbart EP 2345400A2 ein Kit, das die zuvor angeführte Zusammensetzung C2, ein Blondierpulver und eine Oxidationsmittelzusammensetzung im Gewichtsverhältnis von 5 : 1 : 5 enthält. WO 2004/055151A1 offenbart eine flüssige, visuell mindestens zweischichtige Zusammensetzung, die Wasser, Öl und Emulgator enthält, wobei mindestens eine kontinuierliche wässrige Phase I und mindestens eine diskontinuierliche ölige Phase II vorliegen, wobei eine ölige Phase II zusammen mit einem Teil einer wässrigen Phase I eine obere Emulsionsschicht und ein weiterer Teil der wässrigen Phase I eine untere wässrige Schicht bilden. Diese Zusammensetzung lässt sich durch Schütteln temporär in eine visuell homogene Emulsion überführen.

Es war daher die Aufgabe der vorliegenden Erfindung, Mittel zum Aufhellen bzw. Blondieren von Haaren bereitzustellen, die das Haar möglichst wenig schädigen bzw. die oxidative Schädigung durch eine hohe Menge an pflegenden Ölen reduzieren und die dabei einfach herzustellen und zu handhaben sind.

Überraschend wurden diese Aufgaben durch den Gegenstand der Ansprüche gelöst. Ein erster Gegenstand der vorliegenden Erfindung ist eine flüssige, zweischichtige Zusammensetzung mit einem pH-Wert im Bereich von 8 bis 12, bevorzugt 8,5 bis 11, enthaltend
- mindestens ein Alkalisierungsmittel, ausgewählt aus Ammoniak, Alkanolaminen und Mischungen hiervon,
- mindestens ein Öl in einer Gesamtmenge von 20 - 60 Gew.-%, bevorzugt 25 - 40 Gew.-%, und
- mindestens ein Tensid in einer Gesamtmenge von 0,5 - 10 Gew.-%, bevorzugt 2 - 8 Gew.-%, wobei mindestens ein nichtionisches Tensid, ausgewählt aus Ethylenoxid-Anlagerungsprodukten an gesättigte oder ungesättigte lineare Fettalkohole mit jeweils 2 bis 30 Mol Ethylenoxid pro Mol Fettalkohol, sowie mindestens ein anionisches Tensid, ausgewählt aus Alkylsulfaten, Alkylethersulfaten und Alkylethercarbonsäuren mit jeweils 10 bis 18 C-Atomen, bevorzugt 12 bis 14 C-Atomen in der Alkylgruppe und bis zu 12 Glykolethergruppen, bevorzugt 2 bis 6 Glykolethergruppen, im Molekül, enthalten ist,
wobei die Zusammensetzung eine kontinuierliche wässrige Phase I und mindestens eine diskontinuierliche ölige Phase II aufweist, wobei im Ruhezustand die ölige Phase II zusammen mit einem Teil einer wässrigen Phase I eine Emulsionsschicht und ein weiterer Teil der wässrigen Phase I eine wässrige Schicht der Zusammensetzung bilden und sich die Zusammensetzung durch mechanische Bewegung reversibel temporär vollständig in eine Emulsion überführen lässt,
wobei die Mengenangaben jeweils auf das Gewicht der Zusammensetzung bezogen sind.

Die vorstehend beschriebene flüssige, zweischichtige Zusammensetzung mit einem pH-Wert im Bereich von 8 bis 12, bevorzugt 8,5 bis 11, gemessen bei 20°C, ist hervorragend als Alkalisierungsmittelkomponente für ein 3-Komponenten-Haaraufhellungsmittel geeignet, denn sie lässt sich sehr einfach mit üblichen pulverförmigen Persalz-Zubereitungen und einer Wasserstoffperoxidzubereitung zu einer homogenen Anwendungsmischung mit optimaler Viskosität im Bereich von 5000 bis 15000 mPas gemessen bei 20°C mit einem Haake-Viskosimeter Typ MV2 mit einer Geschwindigkeit von 8 Umdrehungen/ Minute, vermischen.

Die erfindungsgemäße Zusammensetzung enthält mindestens ein Alkalisierungsmittel, das ausgewählt ist aus Ammoniak, Alkanolaminen und Mischungen hiervon. Bevorzugte Alkanolamine sind ausgewählt aus Monoethanolamin, 2-Amino-2-methylpropanol und Triethanolamin sowie Mischungen hiervon, wobei Monoethanolamin besonders bevorzugt ist. Ein außerordentlich bevorzugtes Alkalisierungsmittel ist Ammoniak

Die erfindungsgemäße Zusammensetzung enthält mindestens ein Alkalisierungsmittel, das bevorzugt ausgewählt ist aus Ammoniak und Monoethanolamin sowie Mischungen dieser Alkalisierungsmittel.

Üblicherweise wird Ammoniak (NH₃) in Form seiner wässrigen Lösung eingesetzt. Wässrige Ammoniak-Lösungen enthalten Ammoniak (NH₃) oft in Konzentrationen von 10 bis 32 Gew.-%. Bevorzugt ist hierbei der Einsatz einer wässrigen Ammoniak-Lösung, die 25 Gew.-% Ammoniak (NH₃) enthält. Bevorzugt ist die Gesamtmenge an Alkalisierungsmitteln so gewählt, dass die Mischung, also das anwendungsbereite Farbveränderungsmittel, einen alkalischen pH-Wert, bevorzugt einen pH-Wert von 8 bis 11,5, besonders bevorzugt einen pH-Wert von 8,5 bis 11, außerordentlich bevorzugt einen pH-Wert von 9,0 bis 10,5, aufweist. Bevorzugt sind Ammoniak und/oder Monoethanolamin in der erfindungsgemäßen Zusammensetzung in Mengen von 0,01 - 10 Gew.-%, bevorzugt von 0,1 - 7,5 Gew.-%, weiter bevorzugt von 0,5 - 5,5 Gew.-% und besonders bevorzugt von 1,5 - 4,5 Gew.-% - jeweils bezogen auf das Gewicht der Zusammensetzung - enthalten.

Neben Ammoniak und Alkanolaminen kann mindestens ein weiteres Alkalisierungsmittel enthalten sein, das ausgewählt ist aus basischen Aminosäuren sowie anorganischen Alkalisierungsmitteln wie (Erd-)Alkalimetallhydroxiden, (Erd-)Alkalimetallmetasilikaten, (Erd-)Alkalimetallsilikaten, (Erd-)Alkalimetallphosphaten und (Erd-)Alkalimetallhydrogenphosphaten. Geeignete anorganische Alkalisierungsmittel sind Natriumhydroxid, Kaliumhydroxid, Natriumsilicat und Natriummetasilicat. Die als erfindungsgemäßes Alkalisierungsmittel einsetzbaren basischen Aminosäuren sind bevorzugt ausgewählt aus der Gruppe, die gebildet wird aus Arginin, Lysin, Ornithin und Histidin, besonders bevorzugt Arginin.

Die erfindungsgemäße Zusammensetzung enthält, jeweils bezogen auf ihr Gewicht, mindestens ein Öl in einer Gesamtmenge von 20 - 60 Gew.-%, bevorzugt 25 - 40 Gew.-%.

Erfindungsgemäß bevorzugte Öle sind ausgewählt aus natürlichen und synthetischen Kohlenwasserstoffen, besonders bevorzugt aus Paraffinölen, C₁₈-C₃₀-Isoparaffinen, insbesondere Isoeicosan, Polyisobutene und Polydecene, die beispielsweise unter der Bezeichnung Emery® 3004, 3006, 3010 oder unter der Bezeichnung Ethylflo® von Albemarle oder Nexbase® 2004G von Nestle erhältlich sind, weiterhin ausgewählt aus C₈-C₁₆-Isoparaffinen, insbesondere aus Isodecan, Isododecan, Isotetradecan und Isohexadecan sowie Mischungen hiervon, sowie 1,3-Di-(2-ethylhexyl)-cyclohexan (erhältlich z. B. unter dem Handelsnamen Cetiol® S von BASF).

Weitere erfindungsgemäß bevorzugte Öle sind ausgewählt aus den Benzoesäureestern von linearen oder verzweigten C8-22-Alkanolen. Besonders bevorzugt sind Benzoesäure-C12-C15-alkylester, z. B. erhältlich als Handelsprodukt Finsolv® TN, Benzoesäureisostearylester, z. B. erhältlich als Handelsprodukt Finsolv® SB, Ethylhexylbenzoat, z. B. erhältlich als Handelsprodukt Finsolv® EB, und Benzoesäureoctyldocecylester, z. B. erhältlich als Handelsprodukt Finsolv® BOD.

Weitere erfindungsgemäß bevorzugte Öle sind ausgewählt aus Fettalkoholen mit 6 - 30 Kohlenstoffatomen, die ungesättigt oder verzweigt und gesättigt oder verzweigt und ungesättigt sind. Die verzweigten Alkohole werden häufig auch als Guerbet-Alkohole bezeichnet, da sie nach der Guerbet-Reaktion erhältlich sind. Bevorzugte Alkoholöle sind 2-Hexyldecanol (Eutanol® G 16), 2-Octyldodecanol (Eutanol® G), 2-Ethylhexylalkohol und Isostearylalkohol.

Weitere bevorzugte Öle sind ausgewählt aus Mischungen aus Guerbetalkoholen und Guerbetalkoholestern, z.B. dem Handelsprodukt Cetiol® PGL (2-Hexyldecanol und 2-Hexyldecyllaurat).

Weitere erfindungsgemäß bevorzugte kosmetische Öle sind ausgewählt aus den Triglyceriden (= Dreifachestern des Glycerins) von linearen oder verzweigten, gesättigten oder ungesättigten, gegebenenfalls hydroxylierten C8-30-Fettsäuren. Besonders bevorzugt kann die Verwendung natürlicher öle, z.B. Amaranthsamenöl, Aprikosenkernöl, Arganöl, Avocadoöl, Babassuöl, Baumwollsaatöl, Borretschsamenöl, Camelinaöl, Distelöl, Erdnussöl, Granatapfelkernöl, Grapefruitsamenöl, Hanföl, Haselnussöl, Holundersamenöl, Johannesbeersamenöl, Jojobaöl, Leinöl, Macadamianussöl, Maiskeimöl, Mandelöl, Marulaöl, Nachtkerzenöl, Olivenöl, Palmöl, Palmkernöl, Paranussöl, Pekannussöl, Pfirsichkernöl Rapsöl, Rizinusöl, Sanddornfruchtfleischöl, Sanddornkernöl, Sesamöl, Sojaöl, Sonnenblumenöl, Traubenkernöl, Walnussöl, Wildrosenöl, Weizenkeimöl, und die flüssigen Anteile des Kokosöls und dergleichen sein. Bevorzugt sind aber auch synthetische Triglyceridöle, insbesondere Capric/ Caprylic Triglycerides, z. B. die Handelsprodukte Myritol® 318, Myritol® 331 (BASF) oder Miglyol® 812 (Hüls) mit unverzweigten Fettsäureresten sowie Glyceryltriisostearin mit verzweigten Fettsäureresten.

Weitere erfindungsgemäß besonders bevorzugte kosmetische Öle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß besonders bevorzugte kosmetische Öle sind ausgewählt aus den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen 2-Hexyldecylstearat (Eutanol® G 16 S), 2-Hexyldecyllaurat, Isodecylneopentanoat, Isononylisononanoat, 2-Ethylhexylpalmitat (Cegesoft® C 24) und 2-Ethylhexylstearat (Cetiol® 868). Ebenfalls bevorzugt sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und Ethylenglycoldipalmitat.

Weitere erfindungsgemäß bevorzugte kosmetische Öle sind ausgewählt aus den Anlagerungsprodukten von 1 bis 5 Propylenoxid-Einheiten an ein- oder mehrwertige C₈₋₂₂-Alkanole, wie Octanol, Decanol, Decandiol, Laurylalkohol, Myristylalkohol und Stearylalkohol, z. B. PPG-2-Myristylether und PPG-3-Myristylether (Witconol® APM).

Weitere erfindungsgemäß bevorzugte kosmetische Öle sind ausgewählt aus den Anlagerungsprodukten von mindestens 6 Ethylenoxid- und/oder Propylenoxid-Einheiten an ein- oder mehrwertige C₃₋₂₂-Alkanole wie Glycerin, Butanol, Butandiol, Myristylalkohol und Stearylalkohol, die gewünschtenfalls verestert sein können, z. B. PPG-14-Butylether (Ucon Fluid® AP), PPG-9-Butylether (Breox® B25), PPG-10-Butandiol (Macol® 57), PPG-15-Stearylether (Arlamol® E) und Glycereth-7-diisononanoat.

Weitere erfindungsgemäß bevorzugte kosmetische Öle sind ausgewählt aus den C₈-C₂₂-Fettalkoholestern einwertiger oder mehrwertiger C₂-C₇-Hydroxycarbonsäuren, insbesondere die Ester der Glycolsäure, Milchsäure, Äpfelsäure, Weinsäure, Citronensäure und Salicylsäure. Solche Ester auf Basis von linearen C_{14/15}-Alkanolen, z. B. C₁₂-C₁₅-Alkyllactat, und von in 2-Position verzweigten C_{12/13}-Alkanolen sind unter dem Warenzeichen Cosmacol® von der Firma Nordmann, Rassmann GmbH & Co, Hamburg, zu beziehen, insbesondere die Handelsprodukte Cosmacol® ESI, Cosmacol® EMI und Cosmacol® ETI.

Weitere erfindungsgemäß bevorzugte kosmetische Öle sind ausgewählt aus den symmetrischen, unsymmetrischen oder cyclischen Estern der Kohlensäure mit C₃₋₂₂-Alkanolen, C₃₋₂₂-Alkandiolen oder C₃₋₂₂-Alkantriolen, z. B. Dicaprylylcarbonat (Cetiol® CC) oder die Ester gemäß der Lehre der DE 19756454 A1, insbesondere Glycerincarbonat.

Weitere kosmetische Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

Weitere kosmetische Öle, die erfindungsgemäß geeignet sind, sind ausgewählt aus den Siliconölen, zu denen z. B. Dialkyl- und Alkylarylsiloxane, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen. Bevorzugt können flüchtige Siliconöle sein, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind. Ebenfalls geeignet sind flüchtige lineare Siliconöle, insbesondere Hexamethyldisiloxan (L₂), Octamethyltrisiloxan (L₃), Decamethyltetrasiloxan (L₄) sowie beliebige Zweier- und Dreiermischungen aus L₂, L₃ und/ oder L₄, bevorzugt solche Mischungen, wie sie z. B. in den Handelsprodukten DC 2-1184, Dow Corning® 200 (0,65 cSt) und Dow Corning® 200 (1,5 cSt) von Dow Corning enthalten sind. Bevorzugte nichtflüchtige Siliconöle sind ausgewählt aus höhermolekularen linearen Dimethylpolysiloxanen, im Handel erhältlich z. B. unter der Bezeichnung Dow Corning® 190, Dow Corning® 200 Fluid mit kinematischen Viskositäten (25°C) im Bereich von 5 - 100 cSt, bevorzugt 5 - 50 cSt oder auch 5 - 10 cSt, und Dimethylpolysiloxan mit einer kinematischen Viskosität (25°C) von etwa 350 cSt.

Es kann erfindungsgemäß außerordentlich bevorzugt sein, Mischungen der vorgenannten Öle einzusetzen.

Bevorzugte erfindungsgemäße Oxidationszusammensetzungen sind dadurch gekennzeichnet, dass das kosmetische Öl ausgewählt ist aus natürlichen und synthetischen Kohlenwasserstoffen, besonders bevorzugt aus Paraffinölen, C₁₈-C₃₀-Isoparaffinen, insbesondere Isoeicosan, Polyisobutene und Polydecene, C₈-C₁₆-Isoparaffinen, sowie 1,3-Di-(2-ethylhexyl)-cyclohexan; den Benzoesäureestern von linearen oder verzweigten C₈₋₂₂-Alkanolen; Fettalkoholen mit 6 - 30 Kohlenstoffatomen, die ungesättigt oder verzweigt und gesättigt oder verzweigt und ungesättigt sind; Triglyceriden von linearen oder verzweigten, gesättigten oder ungesättigten, gegebenenfalls hydroxylierten C₈₋₃₀-Fettsäuren, insbesondere natürlichen Ölen; den Dicarbonsäureestern von linearen oder verzweigten C₂-C₁₀-Alkanolen; den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können; den Anlagerungsprodukten von 1 bis 5 Propylenoxid-Einheiten an ein- oder mehrwertige C₈₋₂₂-Alkanole; den Anlagerungsprodukten von mindestens 6 Ethylenoxid- und/oder Propylenoxid-Einheiten an ein- oder mehrwertige C₃₋₂₂-Alkanole; den C₈-C₂₂-Fettalkoholestern einwertiger oder mehrwertiger C₂-C₇-Hydroxycarbonsäuren; den symmetrischen, unsymmetrischen oder cyclischen Estern der Kohlensäure mit C₃₋₂₂-Alkanolen, C₃₋₂₂-Alkandiolen oder C₃₋₂₂-Alkantriolen; den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen; Siliconölen sowie Mischungen der vorgenannten Substanzen.

Zur verbesserten Mischbarkeit der erfindungsgemäßen Zusammensetzung mit den übrigen Komponenten des Aufhellmittels sowie besseren Anwendungseigenschaften der resultierenden Anwendungsmischung enthält die erfindungsgemäße Zusammensetzung, jeweils bezogen auf ihr Gewicht, mindestens ein Tensid in einer Gesamtmenge von 0,5 - 10 Gew.-%, bevorzugt 2 - 8 Gew.-%, wobei mindestens ein nichtionisches Tensid, ausgewählt aus Ethylenoxid-Anlagerungsprodukten an gesättigte oder ungesättigte lineare Fettalkohole mit jeweils 2 bis 30 Mol Ethylenoxid pro Mol Fettalkohol, sowie mindestens ein anionisches Tensid, ausgewählt aus Alkylsulfaten, Alkylethersulfaten und Alkylethercarbonsäuren mit jeweils 10 bis 18 C-Atomen, bevorzugt 12 bis 14 C-Atomen in der Alkylgruppe und bis zu 12 Glykolethergruppen, bevorzugt 2 bis 6 Glykolethergruppen, im Molekül, enthalten ist.
Tenside und Emulgatoren im Sinne der vorliegenden Anmeldung sind amphiphile (bifunktionelle) Verbindungen, die aus mindestens einem hydrophoben und mindestens einem hydrophilen Molekülteil bestehen. Der hydrophobe Rest ist bevorzugt eine Kohlenwasserstoffkette mit 8-28 KohlenstoffAtomen, die gesättigt oder ungesättigt, linear oder verzweigt sein kann. Besonders bevorzugt ist diese C₈-C₂₈-Alkylkette linear. Basiseigenschaften der Tenside und Emulgatoren sind die orientierte Absorption an Grenzflächen sowie die Aggregation zu Mizellen und die Ausbildung von lyotropen Phasen.
Erfindungsgemäß ist eine Mischung aus mindestens einem nichtionischen Tensid, ausgewählt aus Ethylenoxid-Anlagerungsprodukten an gesättigte oder ungesättigte lineare Fettalkohole mit jeweils 2 bis 30 Mol Ethylenoxid pro Mol Fettalkohol, sowie mindestens einem anionischen Tensid, ausgewählt aus Alkylsulfaten, Alkylethersulfaten und Alkylethercarbonsäuren mit jeweils 10 bis 18 C-Atomen, bevorzugt 12 bis 14 C-Atomen in der Alkylgruppe und bis zu 12 Glykolethergruppen, bevorzugt 2 bis 6 Glykolethergruppen, im Molekül, besonders geeignet.
Überraschend wurde festgestellt, dass erfindungsgemäße Zusammensetzungen zweischichtig vorliegen mit einer unteren wässrigen Schicht, die im Wesentlichen transparent ist, und einer darüberfe liegenden Emulsionsschicht. Die erfindungsgemäße Zusammensetzung weist eine kontinuierliche wässrige Phase I und mindestens eine diskontinuierliche ölige Phase II auf, wobei im Ruhezustand die ölige Phase II zusammen mit einem Teil einer wässrigen Phase I eine Emulsionsschicht und ein weiterer Teil der wässrigen Phase I eine wässrige Schicht der Zusammensetzung bilden und sich die Zusammensetzung durch mechanische Bewegung reversibel temporär vollständig in eine Emulsion überführen lässt.

Die Tensidmenge reicht also aus, um die Ölmenge komplett zu emulgieren; allerdings verteilt sich die diskontinuierliche Ölphase nicht in der gesamten äußeren Wasserphase, sondern nur in einem Teil hiervon. So gelingt es, die Emulsion trotz eines recht hohen Ölgehalts relativ niedrig-viskos einzustellen, was zu einer hervorragenden Mischbarkeit mit den flüssigen und pulverförmigen Oxidationsmittelkomponenten führt.

Geeignete anionische Tenside sind Alkylsulfate, Alkylethersulfate und Alkylethercarbonsäuren mit jeweils 10 bis 18 C-Atomen, bevorzugt 12 bis 14 C-Atomen in der Alkylgruppe und bis zu 12 Glykolethergruppen, bevorzugt 2 bis 6 Glykolethergruppen im Molekül. Beispiele solcher Tenside sind die Verbindungen mit den INCI-Bezeichnungen Sodium Laureth Sulfate, Sodium Lauryl Sulfate, Sodium Myreth Sulfate oder Sodium Laureth Carboxylate.

Erfindungsgemäße Zusammensetzungen sind dadurch gekennzeichnet, dass mindestens ein nichtionisches Tensid, ausgewählt aus Ethylenoxid-Anlagerungsprodukten an gesättigte oder ungesättigte lineare Fettalkohole mit jeweils 2 bis 30 Mol Ethylenoxid pro Mol Fettalkohol, sowie mindestens ein anionisches Tensid, ausgewählt aus Alkylsulfaten, Alkylethersulfaten und Alkylethercarbonsäuren mit jeweils 10 bis 18 C-Atomen, bevorzugt 12 bis 14 C-Atomen in der Alkylgruppe und bis zu 12 Glykolethergruppen, bevorzugt 2 bis 6 Glykolethergruppen, im Molekül, enthalten ist, wobei bevorzugt das Gewichtsverhältnis der Gesamtheit aller anionischen Tenside zur Gesamtheit aller nichtionischen Tenside im Bereich von 5 - 50, bevorzugt 10 - 30 liegt.
Als optionalen Inhaltsstoff enthält die erfindungsgemäße Zusammensetzung mindestens ein Oxidationsfarbstoffvorprodukt, das vorzugsweise aus einer oder mehreren Entwicklerkomponenten und gegebenenfalls einer oder mehreren Kupplerkomponenten ausgewählt ist.
Bevorzugt ist mindestens ein Oxidationsfarbstoffvorprodukt in einer Gesamtmenge von 0,0001 bis 10,0 Gew.-%, bevorzugt 0,001 bis 8 Gew.-%, jeweils bezogen auf das Gewicht der erfindungsgemäßen Zusammensetzung, enthalten.
Es kann erfindungsgemäß bevorzugt sein, als Entwicklerkomponente mindestens eine Verbindung aus der Gruppe auszuwählen, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin sowie deren physiologisch verträglichen Salzen.
Bevorzugt ist mindestens eine Entwicklerkomponente in einer Gesamtmenge von 0,0001 bis 10,0 Gew.-%, bevorzugt 0,001 bis 8 Gew.-%, jeweils bezogen auf das Gewicht der Zusammensetzung, enthalten.
Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Entwicklerkomponente zusätzlich mindestens eine Kupplerkomponente zum Einsatz kommt.
Erfindungsgemäß bevorzugte Kupplerkomponenten sind ausgewählt aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2-Hydroxyethyl)amino-2-methylphenol, 3-(Diethylamino)phenol, N-Cyclopentyl-3-aminophenol, 1,3-Dihydroxy-5-(methylamino)benzol, 3-Ethylamino-4-methylphenol, 2,4-Dichlor-3-aminophenol, 2-(2,4-Diaminophenoxy)ethanol, 1,3-Bis-(2,4-diaminophenoxy)propan, 1-Methoxy-2-amino-4-(2-hydroxyethylamino)benzol, 1,3-Bis-(2,4-diaminophenyl)propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, Resorcinmonomethylether, 2-Methylresorcin, 5-Methylresorcin, 2,5-Dimethylresorcin, 2-Chlorresorcin, 4-Chlorresorcin, Pyrogallol, 1,2,4-Trihydroxybenzol, 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 2,6-Dihydroxy-3,4-dimethylpyridin, 3,4-Diaminopyridin, 2-(2-Methoxyethyl)amino-3-amino-6-methoxypyridin, 2-(4'-Methoxyphenyl)amino-3-aminopyridin, 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin, 2,3-Dihydroxynaphthalin, 4-Hydroxyindol, 6-Hydroxyindol, 7-Hydroxyindol, 4-Hydroxyindolin, 6-Hydroxyindolin, 7-Hydroxyindolin, 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin oder Gemischen dieser Verbindungen oder deren physiologisch verträglichen Salzen.

Bevorzugt ist mindestens eine Kupplerkomponente in einer Gesamtmenge von 0,0001 bis 10,0 Gew.-%, bevorzugt 0,001 bis 8 Gew.-%, jeweils bezogen auf das Gewicht der Zusammensetzung, enthalten.
Dabei werden Entwicklerkomponenten und Kupplerkomponenten im Allgemeinen in etwa äquimolaren Mengen zueinander eingesetzt. Wenn sich auch der äquimolare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, enthalten sein können.

Weitere erfindungsgemäß bevorzugte Zusammensetzungen sind dadurch gekennzeichnet, dass mindestens ein lineares gesättigtes 1-Alkanol mit 12 - 30 Kohlenstoffatomen enthalten ist, bevorzugt in einer Gesamtmenge von 0,1 - 8 Gew.-%, besonders bevorzugt 3,0 bis 6,0 Gew.-%, jeweils bezogen auf das Gewicht der erfindungsgemäßen Zusammensetzung.
Bevorzugt ist das mindestens eine lineare gesättigte 1-Alkanol mit 12 - 30 Kohlenstoffatomen ausgewählt aus Laurylalkohol, Myristylalkohol, Cetylalkohol, Stearylalkohol, Arachidylalkohol, und Behenylalkohol sowie aus Mischungen dieser Alkanole, besonders bevorzugt aus Cetylalkohol, Stearylalkohol und Cetylalkohol/ Stearylalkohol-Mischungen.
Bevorzugte erfindungsgemäße erfindungsgemäß enthalten, jeweils bezogen auf ihr Gewicht, mindestens ein lineares gesättigtes 1-Alkanol mit 12 - 30 Kohlenstoffatomen in einer Gesamtmenge von 0,1 - 8 Gew.-%, bevorzugt in einer Gesamtmenge von 3,0 - 6,0 Gew.-%, wobei mindestens ein 1-Alkanol, ausgewählt aus Cetylalkohol, Stearylalkohol und Cetylalkohol/ Stearylalkohol-Mischungen, enthalten ist.

Ein weiterer Gegenstand der vorliegenden Erfindung ist eine Mehrkomponentenverpackungseinheit (Kit-of-Parts) zur Aufhellung von keratinischen Fasern, welche mindestens drei getrennt voneinander verpackte Komponenten enthält und welche dadurch gekennzeichnet ist, dass
i) die erste Komponente (I) eine Zusammensetzung nach einem der Ansprüche 1 - ist,
ii) die zweite Komponente (II) pulverförmig vorliegt und mindestens ein Persulfatsalz enthält, und
iii) die dritte Komponente (III), jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid enthält und einen pH-Wert im Bereich von 2,5 bis 5,5, gemessen bei 20°C, aufweist,
wobei die Komponenten (I), (II) und (III) bevorzugt in einem gewichtsbezogenen Verhältnis (I) : (II) : (III) von (2 - 3) : (2 - 3) : (0,7 - 1,3), besonders bevorzugt (2,3 - 2,7) : (2,3 - 2,7) : (0,8 - 1,2) außerordentlich bevorzugt 2,5:2,5:1, vorliegen.

Unter keratinhaltigen bzw. keratinischen Fasern werden erfindungsgemäß Pelze, Wolle, Federn und insbesondere menschliche Haare verstanden. Obwohl die erfindungsgemäßen Mittel in erster Linie zum Blondieren und/oder Aufhellen von keratinhaltigen Fasern geeignet sind, steht prinzipiell einer Verwendung auch auf anderen Gebieten nichts entgegen.

Eine Mehrkomponentenverpackungseinheit umfasst mehrere Einzelkomponenten, die getrennt voneinander konfektioniert sind, sowie eine gemeinsame Verpackung für diese Komponenten, wie zum Beispiel eine Faltschachtel. Darin werden die Komponenten jeweils getrennt in verschiedenen Containern bereitgestellt. Unter Container wird im Rahmen der vorliegenden Erfindung eine Umhüllung verstanden, die in Form einer gegebenenfalls wieder-verschließbaren Flasche, einer Tube, einer Dose, eines Tütchens, eines Sachets oder einer ähnlichen Umhüllung vorliegt. Dem Umhüllungsmaterial sind erfindungsgemäß keine Grenzen gesetzt. Bevorzugt handelt es sich jedoch dabei um Umhüllungen aus Glas oder Kunststoff.
Darüber hinaus kann die Verpackungseinheit Applikationshilfen, wie Kämme, Bürsten oder Pinsel, persönliche Schutzkleidung, insbesondere Ein-Weg-Handschuhe, sowie eine Gebrauchsanleitung umfassen.
Unter dem Begriff "pulverförmig" ist erfindungsgemäß eine feste, rieselfähige Darreichungsform aus einzelnen Partikeln zu verstehen, bei der die einzelnen Partikel Korngrößen im Bereich von 0,1 µm bis maximal 1 mm aufweisen. Gegebenenfalls können die Partikel durch physikalische Behandlung, wie Sieben, Verpressen, Granulieren oder Pelletieren, oder durch den Zusatz bestimmter Hilfsstoffe in ihrer Korngröße den Anforderungen der Mittel angepasst werden, um beispielsweise eine bessere Mischbarkeit der Persulfatsalze zu ermöglichen.
Erfindungsgemäß bevorzugte pulverförmige Persulfatzubereitungen weisen eine Schüttdichte im Bereich von 300 bis 700 g/l (Gramm/Liter), bevorzugt 400 bis 600 g/l, besonders bevorzugt 450 bis 500 g/l, auf. Die Bestimmung der Schüttdichte erfolgt bevorzugt nach aktuell gültiger DIN-Vorschrift. Die pulverförmige Komponente (II) des erfindungsgemäßen Kits ist dadurch gekennzeichnet, dass sie als wesentlichen Bestandteil mindestens ein Persulfatsalz enthält. Vorzugsweise ist dieses Persulfatsalz ausgewählt aus Ammoniumperoxodisulfat, Alkalimetallperoxodisulfaten, Ammoniumperoxomonosulfat und Alkalimetallhydrogenperoxomonosulfaten. Besonders bevorzugt sind Ammoniumperoxodisulfat, Kaliumperoxodisulfat, Natriumperoxodisulfat und Kaliumhydrogenperoxomonosulfat. Weiterhin hat es sich bei den Arbeiten zur vorliegenden Erfindung als besonders bevorzugt erwiesen, wenn die Komponente (II) des erfindungsgemäßen Kits mindestens zwei verschiedene Peroxodisulfate enthält. Bevorzugte Peroxodisulfatsalze sind dabei Kombinationen aus Ammoniumperoxodisulfat und Kaliumperoxodisulfat und/oder Natriumperoxodisulfat.
Eine Ausführungsform des ersten Erfindungsgegenstands ist daher eine Mehrkomponentenverpackungseinheit, die dadurch gekennzeichnet ist, dass die Komponente (II) mindestens Kaliumperoxodisulfat und/oder Ammoniumperoxodisulfat und/oder Natriumperoxodisulfat und/oder mindestens eine Kombination aus Ammoniumperoxodisulfat und Natriumperoxodisulfat und/oder eine Kombination aus Ammoniumperoxodisulfat und Kaliumperoxodisulfat enthält.
Bevorzugt enthält die Komponente (II) ein oder mehrere Persulfatsalze in einer Gesamtmenge von 20 bis 100 Gew.-%, bevorzugt 40 bis 85 Gew.-% und besonders bevorzugt von 50 bis 75 Gew.-%, jeweils bezogen auf das Gewicht der Komponente (II).
Aus Gründen der Lagerstabilität kann die Komponente (II) vorteilhafterweise zusätzlich Stabilisatoren, Alkalitätsgeber, Quellmittel, pflanzliche Gummen, fein gemahlene mineralische Produkte wie Magnesiumcarbonat, Magnesiumoxid oder Bariumoxid und/oder Löslichkeitsverbesserer enthalten. Ebenso können feste Alkalisierungsmittel, wie Alkalimetallcarbonate und insbesondere Alkalimetallsilikate und -metasilikate, in die Komponente (II) eingearbeitet werden. Zur Entstaubung der Pulver können der Komponente (II) entsprechende Entstaubungsmittel, insbesondere Öle, wie Paraffin, Siliconöle oder Esteröle, zugesetzt werden.
Das erfindungsgemäße Kit umfasst als dritte, getrennt konfektioniert vorliegende Komponente (III) eine Formulierung, die Wasserstoffperoxid enthält. Erfindungsgemäß wird Wasserstoffperoxid in dem erfindungsgemäßen Kit als wässrige Wasserstoffperoxid-Lösung eingesetzt.
Die Konzentration einer Wasserstoffperoxid-Lösung wird einerseits von den gesetzlichen Vorgaben und andererseits von dem gewünschten Effekt bestimmt. Sie beträgt 0,5 - 20 Gew.-% Wasserstoffperoxid, bezogen auf das Gewicht der Komponente (III). Erfindungsgemäß bevorzugte Komponenten (III) sind dadurch gekennzeichnet, dass sie, bezogen auf ihr Gewicht, 3-12 Gew.-%, besonders bevorzugt 6 - 9 Gew.-% Wasserstoffperoxid (berechnet als 100%iges H₂O₂), enthalten.
Die Komponente (III) besitzt zur Stabilisierung des Wasserstoffperoxids bevorzugt einen sauren pH-Wert, insbesondere einen pH-Wert von pH-Wert im Bereich von 2,5 bis 5,5, gemessen bei 20°C.

Ein dritter Gegenstand der vorliegenden Erfindung ist ein Verfahren zur Aufhellung von keratinischen Fasern, insbesondere menschlichen Haaren, welches dadurch gekennzeichnet ist, dass die Komponente (I), entsprechend der erfindungsgemäßen Zusammensetzung nach einem der Ansprüche 1 - 4, die Komponente (II) und die Komponente (III) der Mehrkomponentenverpackungseinheit (Kit-of-Parts) gemäß Anspruch 5 oder 6 miteinander vermischt werden, unmittelbar danach auf die keratinhaltigen Fasern aufgebracht, 5 bis 60 Minuten auf den Fasern belassen und anschließend die Fasern mit Wasser gespült und optional mit einem tensidhaltigen Reinigungsmittel ausgewaschen werden, wobei die Komponenten (I), (II) und (III) bevorzugt in einem gewichtsbezogenen Verhältnis (I) : (II) : (III) von (2 - 3) : (2 - 3) : (0,7 - 1,3), besonders bevorzugt (2,3 - 2,7) : (2,3 - 2,7) : (0,8 - 1,2) außerordentlich bevorzugt 2,5:2,5:1, miteinander vermischt werden.
Die anwendungsbereiten Mischungen aus den Komponenten (I), (II) und (III) besitzen eine Viskosität im Bereich von im Bereich von 5000 bis 15000 mPas gemessen bei 20°C mit einem Haake-Viskosimeter Typ MV2 mit einer Geschwindigkeit von 8 Umdrehungen/ Minute, die es ermöglicht, dass das Mittel sich einerseits gut auftragen lässt und andererseits über ein solches Fließverhalten verfügt, dass es für das Mittel eine ausreichend lange Einwirkzeit am Wirkort garantiert.
Bevorzugt beträgt die Einwirkzeit 5 bis 60 min, insbesondere 5 bis 50 min, besonders bevorzugt 10 bis 45 min. Während der Einwirkzeit der Mittel auf der Faser kann es vorteilhaft sein, den Aufhellvorgang durch Wärmezufuhr zu unterstützen. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Die Mittel ergeben bereits bei physiologisch verträglichen Temperaturen von unter 45°C gute Behandlungsergebnisse.

Nach Ende der Einwirkzeit wird das verbleibende Mittel mit einer wässrigen Zubereitung, insbesondere mit Wasser selbst oder einem tensidhaltigen Reinigungsmittel aus dem Haar gespült. Als Reinigungsmittel kann dabei insbesondere ein handelsübliches Shampoo dienen, wobei insbesondere dann auf das Reinigungsmittel verzichtet werden kann und der Ausspülvorgang mit Leitungswasser erfolgen kann, wenn die Anwendungszubereitung ein stark tensidhaltiges Trägermedium besitzt.
Nach Ende des Blondiervorgangs werden alle auf den Keratinfasern befindlichen Komponenten mit Wasser oder einem tensidhaltigen Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Leitungswasser erfolgen kann, wenn das Aufhellmittel einen stark tensidhaltigen Träger besitzt.

Das zu den erfindungsgemäßen und erfindungsgemäß bevorzugten Zusammensetzungen Gesagte gilt mutatis mutandis auch für die erfindungsgemäßen Kits.
Das zu den erfindungsgemäßen und erfindungsgemäß bevorzugten Zusammensetzungen und Kits Gesagte gilt mutatis mutandis auch für die erfindungsgemäßen Verfahren.

### BEISPIELE

### 1. Formulierungen

### (soweit nicht anders angegeben entsprechen die Mengenangaben Gewichtsanteilen)

### 1.1 Herstellung der Komponente (I)

Es wurde die folgende erfindungsgemäße zweischichtige Zusammensetzung hergestellt:

| Inhaltsstoff | Menge (Gew.-%) |
|---|---|
| Kokosfettalkohol | 3,0 |
| Isopropylmyristat | 30,0 |
| Cetearylalkohol | 2,75 |
| Ceteareth-20 | 0,25 |
| Natriumlaurethsulfat (2EO) | 7,0 |
| Ammoniumsulfat | 0,5 |
| Etidronic acid | 0,1 |
| Ammoniak | 1,9 |
| Natriumsilikat 40/42 (Wasserglas) | 0,5 |
| Parfüm | 0,5 |
| Wasser | ad 100,0 |

Kokosfettalkohol und Cetearylalkohol wurden zusammen bei 80 °C aufgeschmolzen, dann wurden Natriumlaurethsulfat (2EO), Isopropylmyristat und Etidronic acid der Reihe nach unter Rühren eingearbeitet. Natriumsilikat 40/42 und Ammoniumsulfat wurden jeweils in einer kleinen Menge Wasser vorgelöst und ebenfalls unter Rühren hinzugefügt. Bei einer Temperatur von ca. 40 °C wurde schließlich der Ammoniak (als wässrige Lösung) zugegeben. Unter Rühren wurde mit Wasser auf 100 Gew.-% aufgefüllt und die Formulierung kalt gerührt.
Die so erhaltene Zusammensetzung lag als flüssige zweischichtig Zusammensetzung vor, wobei die Zusammensetzung eine kontinuierliche wässrige Phase I und eine diskontinuierliche Öl-/Fettphase Phase II aufwies, wobei im Ruhezustand die ölige Phase II zusammen mit einem Teil einer wässrigen Phase I eine obere Schicht in Form einer Öl-in-Wasser-Emulsion und der restliche Teil der wässrigen Phase I eine untere wässrige Schicht der Zusammensetzung bildeten. Durch Schütteln lag temporär die komplette Zusammensetzung als Emulsion vor. Nach 15 Minuten Stehenlassen bildeten sich wieder zwei sichtbare Schichten aus.

### 1.2. Herstellung der Komponente (II)

| Inhaltsstoff | Menge (Gew.-%) |
|---|---|
| Ammoniumpersulfat + 4 % Silica | 5,0 |
| Kaliumpersulfat | 50,0 |
| Natriumpersulfat | 23,0 |
| Natriummetasilikat | 22,0 |

### 1.3 Herstellung der Komponente (III) (Entwickleremulsion)

| Inhaltsstoff | Menge (Gew.-%) |
|---|---|
| Dipicolinsäure | 0,1 |
| Kaliumhydroxid | 0,15 |
| Etidronic acid | 0,2 |
| Natriumcetearylsulfat | 2,5 |
| Cetearylalkohol | 0,5 |
| Ceteareth-20 | 0,5 |
| Bienenwachs | 0,3 |
| Isopropylmyristat | 5,0 |
| Wasserstoffperoxid | 12,0 |
| Wasser | ad 100 |

Die Komponenten (I), (II) und (III) wurden im Gewichtsverhältnis 50:50:20 miteinander vermischt.

### 2. Anwendung

100 g der frisch hergestellten Mischung aus den Komponenten (I), (II) und (III) wurden auf Haarsträhnen appliziert (4 g Anwendungsmischung pro Gramm Haar). Nachdem die Strähnen für 30 min bei 32 °C blondiert wurden, wurden sie mit einem handelsüblichen Shampoo gewaschen und mit einem Föhn getrocknet.

## Patentansprüche

1. Flüssige, zweischichtige Zusammensetzung mit einem pH-Wert im Bereich von 8 bis 12, bevorzugt 8,5 bis 11, enthaltend
- mindestens ein Alkalisierungsmittel, ausgewählt aus Ammoniak, Alkanolaminen und Mischungen hiervon,
- mindestens ein Öl in einer Gesamtmenge von 20 - 60 Gew.-%, bevorzugt 25 - 40 Gew.-%, und
- mindestens ein Tensid in einer Gesamtmenge von 0,5 - 10 Gew.-%, bevorzugt 2 - 8 Gew.-%, wobei mindestens ein nichtionisches Tensid, ausgewählt aus Ethylenoxid-Anlagerungsprodukten an gesättigte oder ungesättigte lineare Fettalkohole mit jeweils 2 bis 30 Mol Ethylenoxid pro Mol Fettalkohol, sowie mindestens ein anionisches Tensid, ausgewählt aus Alkylsulfaten, Alkylethersulfaten und Alkylethercarbonsäuren mit jeweils 10 bis 18 C-Atomen, bevorzugt 12 bis 14 C-Atomen in der Alkylgruppe und bis zu 12 Glykolethergruppen, bevorzugt 2 bis 6 Glykolethergruppen, im Molekül, enthalten ist
wobei die Zusammensetzung eine kontinuierliche wässrige Phase I und mindestens eine diskontinuierliche ölige Phase II aufweist, wobei im Ruhezustand die ölige Phase II zusammen mit einem Teil einer wässrigen Phase I eine Emulsionsschicht und ein weiterer Teil der wässrigen Phase I eine wässrige Schicht der Zusammensetzung bilden und sich die Zusammensetzung durch mechanische Bewegung reversibel temporär vollständig in eine Emulsion überführen lässt,
wobei die Mengenangaben jeweils auf das Gewicht der Zusammensetzung bezogen sind.

2. Zusammensetzung nach Anspruch 1, **dadurch gekennzeichnet, dass** das mindestens eine Öl ausgewählt ist aus natürlichen und synthetischen Kohlenwasserstoffen, besonders bevorzugt aus Paraffinölen, C₁₈-C₃₀-Isoparaffinen, insbesondere Isoeicosan, Polyisobutene und Polydecene, C₈-C₁₆-Isoparaffinen, sowie 1,3-Di-(2-ethylhexyl)-cyclohexan; den Benzoesäureestern von linearen oder verzweigten C₈₋₂₂-Alkanolen; Fettalkoholen mit 6 - 30 Kohlenstoffatomen, die ungesättigt oder verzweigt und gesättigt oder verzweigt und ungesättigt sind; Triglyceriden von linearen oder verzweigten, gesättigten oder ungesättigten, gegebenenfalls hydroxylierten C₈₋₃₀-Fettsäuren; insbesondere natürlichen Ölen; den Dicarbonsäureestern von linearen oder verzweigten C₂-C₁₀-Alkanolen; den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können; den Anlagerungsprodukten von 1 bis 5 Propylenoxid-Einheiten an ein- oder mehrwertige C₈₋₂₂-Alkanole; den Anlagerungsprodukten von mindestens 6 Ethylenoxid- und/oder Propylenoxid-Einheiten an ein- oder mehrwertige C₃₋₂₂-Alkanole; den C₈-C₂₂-Fettalkoholestern einwertiger oder mehrwertiger C₂-C₇-Hydroxycarbonsäuren; den symmetrischen, unsymmetrischen oder cyclischen Estern der Kohlensäure mit C₃₋₂₂-Alkanolen, C₃₋₂₂-Alkandiolen oder C₃₋₂₂-Alkantriolen; den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen; Siliconölen sowie Mischungen der vorgenannten Substanzen.

3. Zusammensetzung nach einem der Ansprüche 1 oder 2, **dadurch gekennzeichnet, dass** das Gewichtsverhältnis der Gesamtheit aller anionischen Tenside zur Gesamtheit aller nichtionischen Tenside im Bereich von 5 - 50, bevorzugt 10 - 30 liegt.

4. Zusammensetzung nach einem der Ansprüche 1 - 3, **dadurch gekennzeichnet, dass** mindestens ein lineares gesättigtes 1-Alkanol mit 12 - 30 Kohlenstoffatomen enthalten ist, bevorzugt in einer Gesamtmenge von 0,1 - 8 Gew.-%, besonders bevorzugt 3,0 bis 6,0 Gew.-%, jeweils bezogen auf das Gewicht der Zusammensetzung.

5. Mehrkomponentenverpackungseinheit (Kit-of-Parts) zur Aufhellung von keratinischen Fasern, enthaltend mindestens drei getrennt voneinander verpackte Komponenten, **dadurch gekennzeichnet, dass**
i) die erste Komponente (I) eine Zusammensetzung nach einem der Ansprüche 1 - 4 ist,
ii) die zweite Komponente (II) pulverförmig vorliegt und mindestens ein Persulfatsalz enthält, und
iii) die dritte Komponente (III), jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid enthält und einen pH-Wert im Bereich von 2,5 bis 5,5, gemessen bei 20°C, aufweist,
wobei die Komponenten (I), (II) und (III) bevorzugt in einem gewichtsbezogenen Verhältnis (I) : (II) : (III) von (2 - 3) : (2 - 3) : (0,7 - 1,3), besonders bevorzugt (2,3 - 2,7) : (2,3 - 2,7) : (0,8 - 1,2) außerordentlich bevorzugt 2,5:2,5:1, vorliegen.

6. Mehrkomponentenverpackungseinheit gemäß Anspruch 5, **dadurch gekennzeichnet, dass** die Komponente (II) mindestens Kaliumperoxodisulfat und/oder Ammoniumperoxodisulfat und/oder Natriumperoxodisulfat und/oder mindestens eine Kombination aus Ammoniumperoxodisulfat und Natriumperoxodisulfat und/oder eine Kombination aus Ammoniumperoxodisulfat und Kaliumperoxodisulfat enthält, bevorzugt in einer Gesamtmenge von 20 bis 100 Gew.-%, bevorzugt 40 bis 85 Gew.-% und besonders bevorzugt von 50 bis 75 Gew.-%, jeweils bezogen auf das Gewicht der Komponente (II).

7. Verfahren zur Aufhellung von keratinischen Fasern, insbesondere menschlichen Haaren, welches **dadurch gekennzeichnet ist, dass** die Komponente (I), entsprechend der Zusammensetzung nach einem der Ansprüche 1 - 4, die Komponente (II) und die Komponente (III) der Mehrkomponentenverpackungseinheit (Kit-of-Parts) gemäß Anspruch 5 oder 6 miteinander vermischt werden, unmittelbar danach auf die keratinhaltigen Fasern aufgebracht, 5 bis 60 Minuten auf den Fasern belassen und anschließend die Fasern mit Wasser gespült und optional mit einem tensidhaltigen Reinigungsmittel ausgewaschen werden,
wobei die Komponenten (I), (II) und (III) bevorzugt in einem gewichtsbezogenen Verhältnis (I) : (II) : (III) von (2 - 3) : (2 - 3) : (0,7 - 1,3), besonders bevorzugt (2,3 - 2,7) : (2,3 - 2,7) : (0,8 - 1,2) außerordentlich bevorzugt 2,5:2,5:1, miteinander vermischt werden.

## Claims

1. A liquid two-layered composition having a pH value in the range of 8 to 12, preferably 8.5 to 11, containing
- at least one alkalizing agent selected from ammonia, alkanolamines and mixtures thereof,
- at least one oil in a total amount of 20 - 60 wt.-%, preferably 25 - 40 wt.-%, and
- at least one surfactant in a total amount of 0.5 - 10 wt.-%, preferably 2 - 8 wt.-%, wherein at least one nonionic surfactant selected from ethylene oxide addition products to saturated or unsaturated linear fatty alcohols each having from 2 to 30 moles of ethylene oxide per mole of fatty alcohol, and at least one anionic surfactant, selected from alkyl sulfates, alkyl ether sulfates and alkyl ether carboxylic acids each having 10 to 18 carbon atoms, preferably 12 to 14 carbon atoms in the alkyl group and up to 12 glycol ether groups, preferably 2 to 6 glycol ether groups, in the molecule, is contained,
wherein the composition comprises a continuous aqueous phase I and at least one discontinuous oily phase II, wherein in the rest state the oily phase II together with a part of an aqueous phase I form an emulsion layer and a further part of the aqueous phase I form an aqueous layer of the composition and wherein the composition can be reversibly temporarily completely converted into an emulsion by mechanical movement,
wherein the quantities are each based on the weight of the composition.

2. The composition according to claim 1, **characterized in that** the at least one oil is selected from natural and synthetic hydrocarbons, particularly preferably from paraffin oils, C₁₈-C₃₀-isoparaffins, in particular isoeicosan, polyisobutenes and polydecenes, C₈-C₁₆-isoparaffins, and 1,3-di-(2-ethylhexyl)-cyclohexane; the benzoic acid esters of linear or branched C₈-₂₂-alkanols; fatty alcohols having 6 - 30 carbon atoms which are unsaturated or branched and saturated or branched and unsaturated; triglycerides of linear or branched, saturated or unsaturated, optionally hydroxylated C₈-C₃₀ fatty acids, in particular natural oils; the dicarboxylic esters of linear or branched C₂-C₁₀-alkanols; the esters of linear or branched saturated or unsaturated fatty alcohols with 2-30 carbon atoms with linear or branched saturated or unsaturated fatty acids with 2-30 carbon atoms, which may be hydroxylated; the addition products of 1 to 5 propylene oxide units to monovalent or polyvalent C₈-₂₂-alkanols; the addition products of at least 6 ethylene oxide and/or propylene oxide units to monovalent or polyvalent C₃-₂₂₋alkanols; the C₈-C₂₂ fatty alcohol esters of monovalent or polyvalent C₂-C₇-hydroxycarboxylic acids; the symmetrical, asymmetrical or cyclic esters of carbonic acid with C₃-₂₂-alkanols, C₃-₂₂-alkanediols or C₃-₂₂-alkanetriols; the esters of dimers of unsaturated C₁₂-C₂₂ fatty acids (dimer fatty acids) with monovalent linear, branched or cyclic C₂-C₁₈-alkanols or with polyvalent linear or branched C₂-C₆-alkanols; silicone oils and mixtures of the above mentioned substances.

3. The composition according to one of claims 1 or 2, **characterized in that** the weight ratio of the total of all anionic surfactants to the total of all nonionic surfactants is in the range from 5 - 50, preferably 10 - 30.

4. The composition according to any one of claims 1 to 3, **characterized in that** it contains at least one linear saturated 1-alkanol having 12 - 30 carbon atoms, preferably in a total amount of 0.1 - 8 wt.-%, more preferably 3.0 to 6.0 wt.-%, each based on the weight of the composition.

5. A multi-component packaging unit (kit-of-parts) for bleaching keratinous fibers, containing at least three components packaged separately from one another, **characterized in that**
i) the first component (I) is a composition according to any of claims 1 - 4,
ii) the second component (II) is in powder form and contains at least one persulfate salt, and
iii) the third component (III) contains 50 - 96 wt.-%, preferably 70 - 93 wt.-%, particularly preferably 80 - 90 wt.-%, of water and 0.5 - 20 wt.-% of hydrogen peroxide, based in each case on its weight, and has a pH value in the range from 2.5 to 5.5, measured at 20°C, wherein the components (I), (II) and (III) are preferably present in a weight-related ratio (I) : (II) : (III) of (2 - 3) : (2 - 3) : (0.7 - 1.3), particularly preferably of (2.3 - 2.7) : (2.3 - 2.7) : (0.8 - 1.2) particularly preferably of 2.5:2.5:1.

6. The multi-component packaging unit according to claim 5, **characterized in that** component (II) has at least potassium peroxodisulphate and/or ammonium peroxodisulphate and/or sodium peroxodisulphate and/or at least one combination of ammonium peroxodisulphate and sodium peroxodisulphate and/or a combination of ammonium peroxodisulphate and potassium peroxodisulphate, preferably in a total amount of 20 to 100 wt.-%, preferably of 40 to 85 wt.-% and particularly preferably of 50 to 75 wt.-%, in each case based on the weight of component (II).

7. A method for bleaching keratinous fibers, in particular human hair, which is **characterized in that** the component (I), according to the composition of one of the claims 1 to 4, the component (II) and the component (III) of the multi-component packaging unit (kit-of-parts) according to claim 5 or 6 are mixed together, are applied immediately thereafter to the keratin-containing fibers, left on the fibers for 5 to 60 minutes and then the fibers are rinsed with water and optionally washed out with a surfactant-containing cleaning agent, wherein the components (I), (II) and (III) are preferably mixed together in a weight-related ratio (I) : (II) : (III) of (2 - 3) : (2 - 3) : (0.7 - 1.3), particularly preferably of (2.3 - 2.7) : (2.3 - 2.7) : (0.8 - 1.2) particularly preferably of 2.5:2.5:1.

## Revendications

1. Composition bicouche liquide présentant un pH situé dans la plage allant de 8 à 12, de préférence de 8,5 à 11, et contenant
- au moins un agent alcalinisant choisi parmi l'ammoniac, les alcanolamines et les mélanges de ceux-ci,
- au moins une huile, en une quantité totale située dans la plage allant de 20 à 60 % en poids, de préférence de 25 à 40 % en poids, et
- au moins un agent tensioactif, en une quantité totale d'au moins 0,5 à 10 % en poids, de préférence de 2 à 8 % en poids, au moins un tensioactif non ionique étant présent, choisi parmi les produits d'addition d'oxyde d'éthylène avec des alcools gras linéaires saturés ou insaturés, ayant chacun 2 à 30 moles d'oxyde d'éthylène par mole d'alcool gras, et au moins un tensioactif anionique étant présent, choisi parmi les alkylsulfates, les alkyléthersulfates et les acides alkyléthercarboxyliques ayant chacun de 10 à 18 atomes de carbone, de préférence de 12 à 14 atomes de carbone dans le groupe alkyle et jusqu'à 12 groupes éther de glycol, de préférence de 2 à 6 groupes éther de glycol, dans la molécule,
la composition comportant une phase aqueuse I continue et au moins une phase huileuse II discontinue, la phase huileuse II formant, à l'état de repos, conjointement avec une fraction d'une phase aqueuse I, une couche d'émulsion et, avec une autre fraction de la phase aqueuse I, une couche aqueuse de la composition, et la composition étant intégralement convertie, de manière temporaire et réversible, en émulsion par un mouvement mécanique, les quantités indiquées se rapportant, dans chaque cas, au poids de la composition.

2. Composition selon la revendication 1, **caractérisée en ce que** l'au moins une huile est choisie parmi les hydrocarbures naturels ou synthétiques, de manière particulièrement préférée parmi les huiles de paraffine, les isoparaffines en C₁₈ à C₃₀ en particulier l'isoéicosane, les polyisobutènes et les polydécènes, les isoparaffines en C₈ à C₁₆, ainsi que le 1,3-di-(2-éthylhexyl)-cyclohexane ; les esters d'acide benzoïque d'alcanols en C₈ à C₂₂ linéaires ou ramifiés ; les alcools gras ayant de 6 à 30 atomes de carbone, qui sont insaturés ou ramifiés et saturés ou ramifiés et insaturés ; les tiglycérides d'acides gras en C₈ à C₃₀ linéaires ou ramifiés, saturés ou insaturés, éventuellement hydroxylés, en particulier les huiles naturelles ; les esters d'acide dicarboxylique d'alcanols en C₂ à C₁₀ linéaires ou ramifiés ; les esters d'alcools gras linéaires ou ramifiés, saturés ou insaturés ayant de 2 à 30 atomes de carbone avec des acides gras linéaires ou ramifiés, saturés ou insaturés ayant de 2 à 30 atomes de carbone, qui peuvent être hydroxylés ; les produits d'addition de 1 à 5 unités d'oxyde de propylène à des alcanols en C₈ à C₂₂ mono- ou multivalents ; les produits d'addition d'au moins 6 unités d'oxyde d'éthylène et/ou d'oxyde de propylène à des alcanols en C₃ à C₂₂ mono- ou polyvalents ; les esters d'alcool gras en C₈ à C₂₂ d'acides hydroxycarboxyliques en C₂ à C₇ mono- ou multivalents ; les esters symétriques, asymétriques ou cycliques des acides carboniques avec des alcanols en C₃ à C₂₂, des alcanediols en C₃ à C₂₂ ou des alsanetriols en C₃ à C₂₂ ; les esters de dimères d'acides gras en C₁₂ à C₂₂ insaturés (acides gras dimères) avec des alcanols en C₂ à C₁₈ linéaires, ramifiés ou cycliques monovalents ou avec des alcanols en C₂ à C₆ linéaires ou ramifiés multivalents ; les huiles de silicone ainsi que les mélanges des substances précitées.

3. Composition selon l'une des revendications 1 ou 2, **caractérisée en ce que** le rapport en poids du total des tensioactifs anioniques par rapport au total des tensioactifs non ioniques se situe dans la plage allant de 5 à 50, de préférence de 10 à 30.

4. Composition selon l'une des revendications 1 à 3, **caractérisée en ce qu'**au moins un 1-alcanol saturé linéaire ayant de 12 à 30 atomes de carbone est présent, de préférence en une quantité totale située dans la plage allant de 0,1 à 8 % en poids, de manière particulièrement préférée de 3,0 à 6,0 % en poids, dans chaque cas par rapport au poids de la composition.

5. Unité de conditionnement à composants multiples (« kit ») pour l'éclaircissement des fibres kératiniques, contenant au moins trois composants conditionnés séparément les uns des autres, et **caractérisée en ce que**
i) le premier composant (I) est une composition selon l'une des revendications 1 à 4,
ii) le deuxième composant (II) étant présent sous forme de poudre et contient au moins un sel de persulfate, et
iii) le troisième composant (III) contient, dans chaque cas par rapport à son poids, de 50 à 96 % en poids, de préférence de 70 à 93 % en poids, de manière particulièrement préférée de 80 à 90 % en poids d'eau et de 0,5 à 20 % en poids de peroxyde d'hydrogène et présente un pH situé dans la plage allant de 2,5 à 5,5, mesuré à 20 °C, les composants (I), (II) et (III) étant de préférence présents dans un rapport en poids (I):(II):(III) de (2 à 3) : (2 à 3) : (0,7 à 1,3), de manière particulièrement préférée de (2,3 à 2,7) : (2,3 à 2,7) : (0,8 à 1,2), et de manière préférée entre toutes de 2,5:2,5:1.

6. Unité de conditionnement à composants multiples selon la revendication 5, **caractérisée en ce que** le composant (II) contient au moins du peroxydisulfate de potassium et/ou du peroxydisulfate d'ammonium et/ou du peroxydisulfate de sodium et/ou au moins une combinaison de peroxydisulfate d'ammonium et de peroxydisulfate de sodium et/ou une combinaison de peroxydisulfate d'ammonium et de peroxydisulfate de potassium, de préférence en une quantité totale située dans la plage allant de 20 à 100 % en poids, de préférence de 40 à 85 % en poids et de manière particulièrement préférée de 50 à 75 % en poids, dans chaque cas par rapport au poids du composant (II).

7. Procédé d'éclaircissement des fibres kératiniques, en particulier des cheveux humains, **caractérisé en ce que** le composant (I), correspondant à la composition selon l'une des revendications 1 à 4, le composant (II) et le composant (III) de l'unité de conditionnement à composants multiples (« kit ») selon la revendication 5 ou 6 sont mélangés les uns avec les autres, puis immédiatement appliqués sur les fibres kératiniques, laissés à poser sur les fibres pendant 5 à 60 minutes, puis les fibres sont rincées à l'eau et éventuellement lavées avec un détergent contenant un tensioactif,
les composants (I), (II) et (III) étant de préférence mélangés les uns avec les autres dans un rapport en poids (I) : (II) : (III) de (2 à 3) : (2 à 3) : (0,7 à 1, 3), de manière particulièrement préférée de (2,3 à 2,7) : (2,3 à 2,7) : (0,8 à 1,2), et de manière préférée entre toutes de 2,5:2,5:1.
